# EUROPÄISCHE PATENTSCHRIFT

(11) **EP 1 352 667 B1**
(45) Veröffentlichungstag und Bekanntmachung des Hinweises auf die Patenterteilung: **15.12.2004**
(21) Anmeldenummer: 02008347.3
(22) Anmeldetag: 12.04.2002
(51) Int. Cl.: A61M 5/31, A61M 5/34

(54) **Verfahren zum Herstellen einer Injektionsspritze mit eingeklebter Kanüle**
Method for producing an injection syringe with a glued-in cannula
Procédé de fabrication de seringue d'injection avec canule fixée par collage

(43) Veröffentlichungstag der Anmeldung: 15.10.2003
(73) Patentinhaber: Bünder Glas GmbH, 32257 Bünde (DE)
(72) Erfinder: Brandhorst, Erik, 32120 Hiddenhausen (DE); Geiger, Andreas, 32278 Kirchlengern (DE)
(74) Vertreter: Schirmer, Siegfried, Dipl.-Ing.

(56) Entgegenhaltungen:
- EP-A- 0 675 315
- EP-A- 0 962 229
- DE-A- 3 339 705
- DE-C- 10 036 832

## Beschreibung

Die Erfindung betrifft ein Verfahren zum Herstellen einer Injektionsspritze mit eingeklebter Kanüle.

Injektionsspritzen werden in der Regel auf der Innenfläche des Spritzenkörpers mit einem Gleitmittel behandelt, welches meist bei hoher Temperatur aufgedampft bzw. "eingebrannt" wird, damit sichergestellt ist, daß kein überschüssiges Gleitmittel zusammen mit dem Wirkstoff injiziert wird.

Hieraus ergibt sich die Schwierigkeit, daß bei Spritzen mit eingeklebter Kanüle der Klebstoff, mit dem die Kanüle in eine Kanülenaufnahmeöffnung des Spritzenkörpers eingeklebt wird, hohen Temperaturen (bspw. 250 bis 300 °C) ohne Verfärbung bzw. Zerstörung nicht aushält.

Umgekehrt ist es jedoch auch schwierig, einen bereits mit Gleitmittel behandelten Spritzenkörper mit einer Kanüle zu versehen, da eine mit Gleitmittel behandelte Oberfläche einen Klebstoff nicht mehr ausreichend gut annimmt.

Bei Glasspritzen wird in der Regel eine Silikonemulsion verwendet, mit der die Innenfläche des Spritzenkörpers benetzt wird und anschließend das Silikon in einem Ofen bei etwa 260°C während einer Dauer von 15 bis 120 Minuten eingebrannt wird. Dabei gelangt unvermeidlich auch Silikon auf die Innenwand der Kanülenaufnahmeöffnung, so daß eine sichere Verklebung nicht mehr gewährleistet ist.

Aus der DE 100 36 832 C1 ist ein Verfahren und eine Vorrichtung zum Aufbringen einer hitzefixierten Gleitmittelschicht auf die Innenwandung einer Glasspritze bekannt. Es wird vorgeschlagen, eine bereits mit einer eingeklebten Nadel versehene Glasspritze innen mit einem Gleitmittel zu versehen, beispielsweise durch Aufsprühen. Hierbei kann Gleitmittel teilweise auch in den Bereich der Nadel gelangen. Anschließend wird durch lokale Wärmebeaufschlagung das Gleitmittel in den gewünschten Bereichen eingebrannt, wobei eine derartige Fixierung des Gleitmittels im Bereich der Nadel verhindert wird, damit kein Verstopfen der Nadel eintreten kann. Bei dieser Vorgehensweise besteht der Nachteil, daß nicht kontrolliert werden kann, ob und ggf. in welcher Menge das zur Beschichtung verwendete Gleitmittel in die bereits eingeklebte Nadel gelangt.

Aus der DE 33 39 705 A1 ist bekannt, ein separat von einem Spritzenkörper hergestelltes Nadelansatzstück zu verwenden, in das eine Nadel eingeklebt ist, wobei dann das Nadelansatzstück mit Nadel in den Spritzenkörper verrastend eingesetzt wird, nachdem dieser auf seiner Innenseite mit einem Gleitmittel versehen worden ist. Die Verwendung zahlreicher Einzelteile ist aufwendig im Hinblick auf Herstellung und Handhabung.

Die Erfindung hat sich zur Aufgabe gestellt, ein Verfahren zum Herstellen einer Injektionsspritze mit eingeklebter Kanüle bereitzustellen, bei dem trotz Aufbringung eines Gleitmittels bei hoher Temperatur eine sichere Verklebung der Kanüle möglich ist.

Diese Aufgabe wird erfindungsgemäß durch ein Verfahren zum Herstellen einer Injektionsspritze mit eingeklebter Kanüle gemäß Anspruch 1 gelöst.

Das Gleitmittel kann mit Hilfe eines chemischen Lösungsmittels, mittels Plasmaenergie, mechanisch, abrasiv oder durch Strahlen (z.B. mit Aluminiumoxid-Partikeln) entfernt werden.

In jedem Falle wird gewährleistet, daß die mit dem Klebstoff in Berührung kommende Innenfläche der Kanülenaufnahmeöffnung vor dem Verkleben im wesentlichen von unerwünschtem Gleitmittel befreit wird, so daß eine tragfähige Verklebung zustandekommt.

Die Aufgabe der Erfindung wird weiter gelöst durch ein Verfahren zum Herstellen einer Injektionsspritze mit eingeklebter Kanüle gemäß Anspruch 7.

Bei diesem Aspekt der Erfindung wird somit von vornherein verhindert, daß Gleitmittel in unerwünschter Weise während des Einbrennvorgangs in die Kanülenaufnahmeöffnung eindringt und sich an deren Innenwand ablagert. Dies kann dadurch erfolgen, daß die Kanülenaufnahmeöffnung vorübergehend verschlossen wird, bspw. beidenends mit je einer Membran aus hitzebeständigem Material oder mit einem pfropfenförmigen Verschlußkörper aus hitzebeständigem Material.

Alternativ könnte die Kanülenaufnahmeöffnung mit einem Spülgas, bspw. Luft, Stickstoff oder CO₂, gespült werden, während der Spritzenkörper mit dem Gleitmittel versehen wird.

Bei beiden erfindungsgemäßen Verfahrensweisen kann vorgesehen sein, daß das Gleitmittel aufgedampft wird, oder daß das Gleitmittel in Form einer Emulsion aufgebracht und bei ca. 250 bis 300°C eingebrannt wird. Hierbei kann bevorzugt Silikon als Gleitmittel verwendet werden. Als Klebstoff kann ein herkömmlicherweise verwendeter, nicht hitzebeständiger Klebstoff verwendet werden, z. B. Polyurethanmethacrylat.

Bevorzugt besteht der Spritzenkörper aus Glas. Das Gleitmittel kann Silikon sein. Bevorzugt ist vorgesehen, daß die Kanülenaufnahmeöffnung innen gestrahlt ist.

Die Erfindung wird nachfolgend anhand eines Ausführungsbeispiels erläutert.

Glasspritzen werden mit einer Silikonemulsion (1%) innensilikonisiert und in einem Ofen bei 260°C während 120 Minuten eingebrannt.

Nach dem Einbrandsilikonisieren wird die eingebrannte Silikonschicht aus der Kanülenaufnahmeöffnung durch ein chemisches Verfahren mit Methylenchlorid oder alternativ durch ein mechanisches Verfahren durch Strahlen mit Aluminiumoxid (Körnung: 2/100 mm) entfernt.

Bei der chemischen Entfernung wurden die Spritzen in ein Methylenchloridbad getaucht, wobei die Nadelaufnahmeöffnung vollständig eingetaucht war.

Anschließend wurde die Kanüle mit einem herkömmlichen Klebstoff eingeklebt, und die Spritzen wurden autoklaviert.

Sowohl die chemisch als auch die mechanisch behandelten Spritzen haben eine Kanülenabzugsprüfung bestanden, was bedeutet, daß die erfindungsgemäß erzielte Klebkraft wesentlich größer ist als bei einbrandsilikonisierten und nicht weiter behandelten Spritzen.

## Patentansprüche

1. Verfahren zum Herstellen einer Injektionsspritze mit eingeklebter Kanüle, bei dem folgende Schritte nacheinander ausgeführt werden:
- Aufbringen eines Gleitmittels innen auf einen eine Kanülenaufnahnleöffnung aufweisenden Spritzenkörper bei hoher Temperatur,
- Befreien der Kanülenaufnahmeöffnung von dem Gleitmittel,
- Einkleben einer Kanüle in die Kanülenaufnahmeöffnung.

2. Verfahren nach Anspruch 1, **dadurch gekennzeichnet, daß** das Gleitmittel mit Hilfe eines chemischen Lösungsmittels, insbesondere Methylenchlorid, entfernt wird.

3. Verfahren nach Anspruch 1, **dadurch gekennzeichnet, daß** das Gleitmittel mittels Plasmaenergie entfernt wird.

4. Verfahren nach Anspruch 1, **dadurch gekennzeichnet, daß** das Gleitmittel mechanisch entfernt wird.

5. Verfahren nach Anspruch 4, **dadurch gekennzeichnet, daß** das Gleitmittel abrasiv entfernt wird.

6. Verfahren nach Anspruch 5, **dadurch gekennzeichnet, daß** das Gleitmittel durch Strahlen entfernt wird.

7. Verfahren zum Herstellen einer Injektionsspritze mit eingeklebter Kanüle, bei dem folgende Schritte nacheinander ausgeführt werden:
- Aufbringen eines Gleitmittels innen auf einen eine Kanülenaufnahmeöffnung aufweisenden Spritzenkörper bei hoher Temperatur,
- Verhindern, gleichzeitig mit dem Aufbringen, eines Eindringens von Gleitmittel in die Kanülenaufnahmeöffnung,
- Einkleben einer Kanüle in die Kanülenaufnahmeöffnung.

8. Verfahren nach Anspruch 7, **dadurch gekennzeichnet, daß** die Kanülenaufnahmeöffnung vorübergehend verschlossen wird, so daß kein Gleitmittel in diese eindringen kann.

9. Verfahren nach Anspruch 8, **dadurch gekennzeichnet, daß** die Kanülenaufnahmeöffnung beidenends mit je einer Membran aus hitzebeständigem Material verschlossen wird.

10. Verfahren nach Anspruch 8, **dadurch gekennzeichnet, daß** die Kanülenaufnahmeöffnung beidenends mit einem pfropfenförmigen Verschlußkörper aus hitzebeständigen Material verschlossen wird.

11. Verfahren nach Anspruch 7, **dadurch gekennzeichnet, daß** die Kanülenaufnahmeöffnung, mit einem Spülgas, insbesondere Luft, Stickstoff oder Kohlendioxid, gespült wird, während der Spritzenkörper mit dem Gleitmittel versehen wird.

12. Verfahren nach einem der vorangehenden Ansprüche, **dadurch gekennzeichnet, daß** das Gleitmittel aufgedampft wird.

13. Verfahren nach einem der vorangehenden Ansprüche, **dadurch gekennzeichnet, daß** das Gleitmittel in Form einer Emulsion aufgebracht und bei ca. 250 bis 300°C eingebrannt wird.

14. Verfahren nach Anspruch 13, **dadurch gekennzeichnet, daß** Silikon als Gleitmittel verwendet wird.

15. Verfahren nach einem der vorangehenden Ansprüche, **dadurch gekennzeichnet, daß** ein nicht hitzebeständiger Klebstoff, insbesondere Polyurethanmethacrylat, verwendet wird.

## Claims

1. Method of producing an injection syringe with glued-in cannula, in which the following steps are carried out in succession:
- application of a sliding agent internally to a syringe body, which has a cannula receiving opening, at high temperature,
- freeing the cannula receiving opening from the sliding agent,
- gluing a cannula into the cannula receiving opening.

2. Method according to claim 1, **characterised in that** the sliding agent is removed with the help of a chemical solvent, particularly methylene chloride.

3. Method according to claim 1, **characterised in that** the sliding agent is removed by means of plasma energy.

4. Method according to claim 1, **characterised in that** the sliding agent is mechanically removed.

5. Method according to claim 4, **characterised in that** the sliding agent is abrasively removed.

6. Method according to claim 5, **characterised in that** the sliding agent is removed by blasting.

7. Method of producing an injection syringe with glued-in cannula, in which the following steps are carried out in succession:
- application of a sliding agent internally to a syringe body, which has a cannula receiving opening, at high temperature,
- preventing, simultaneously with the application, penetration of sliding agent into the cannula receiving opening,
- gluing a cannula into the cannula receiving opening.

8. Method according to claim 7, **characterised in that** the cannula receiving opening is temporarily closed so that no sliding agent can penetrate into this.

9. Method according to claim 8, **characterised in that** the cannula receiving opening is closed at each end by a respective membrane of heat-resistant material.

10. Method according to claim 8, **characterised in that** the cannula receiving opening is closed at both ends by a drop-shaped closure body of heat-resistant material.

11. Method according to claim 7, **characterised in that** the cannula receiving opening is washed with a flushing gas, particularly air, nitrogen or carbon dioxide, while the syringe body is provided with the sliding agent.

12. Method according to one of the preceding claims, **characterised in that** the sliding agent is vapour-deposited.

13. Method according to one of the preceding claims, **characterised in that** the sliding agent is applied in the form of an emulsion and is baked at approximately 250 to 300° C.

14. Method according to claim 13, **characterised in that** silicon is used as sliding agent.

15. Method according to one of the preceding claims, **characterised in that** an adhesive, particularly polyurethane methacrylate, which is not heat-resistant is used.

## Revendications

1. Procédé de fabrication d'une seringue à injection avec canule fixée par collage,
**caractérisé par**
les étapes suivantes réalisées l'une après l'autre :
- application d'un lubrifiant à température élevée à l'intérieur sur un corps de seringue présentant une ouverture de réception de canule,
- élimination du lubrifiant de l'ouverture de réception de canule, et
- fixation d'une canule par collage dans l'ouverture de réception de canule.

2. Procédé selon la revendication 1,
**caractérisé en ce que**
le lubrifiant est éliminé à l'aide d'un solvant chimique, en particulier du chlorure de méthylène.

3. Procédé selon la revendication 1,
**caractérisé en ce que**
le lubrifiant est éliminé au moyen d'énergie fournie par du plasma.

4. Procédé selon la revendication 1,
**caractérisé en ce que**
le lubrifiant est éliminé mécaniquement.

5. Procédé selon la revendication 4,
**caractérisé en ce que**
le lubrifiant est éliminé par abrasion.

6. Procédé selon la revendication 5,
**caractérisé en ce que**
le lubrifiant est éliminé par grenaillage.

7. Procédé de fabrication d'une seringue à injection avec canule fixée par collage,
**caractérisé par**
les étapes suivantes l'une après l'autre :
- application d'un lubrifiant à température élevée à l'intérieur sur un corps de seringue présentant une ouverture de réception de canule,
- empêchement, en même temps que l'application, d'une pénétration de lubrifiant dans l'ouverture de réception de canule, et
- fixation d'une canule par collage dans l'ouverture de réception de canule.

8. Procédé selon la revendication 7,
**caractérisé en ce que**
l'ouverture de réception de canule est temporairement fermée, de sorte qu'aucun lubrifiant ne peut y pénétrer.

9. Procédé selon la revendication 8,
**caractérisé en ce que**
l'ouverture de réception de canule est fermée aux deux extrémités, chacune par une membrane en matériau résistant à la chaleur.

10. Procédé selon la revendication 8,
**caractérisé en ce que**
l'ouverture de réception de canule est fermée aux deux extrémités par un corps de fermeture en forme de tampon en matériau résistant à la chaleur.

11. Procédé selon la revendication 7,
**caractérisé en ce que**
l'ouverture de réception de canule est purgée avec un gaz de purge, en particulier de l'air, de l'azote ou du dioxyde de carbone, pendant que le corps de seringue est muni de lubrifiant.

12. Procédé selon l'une quelconque des revendications précédentes,
**caractérisé en ce que**
le lubrifiant est métallisé sous vide.

13. Procédé selon l'une quelconque des revendications précédentes,
**caractérisé en ce que**
le lubrifiant est appliqué sous forme d'une émulsion et cuit à env. 250 à 300°C.

14. Procédé selon la revendication 13,
**caractérisé en ce que**
du silicone est utilisé comme lubrifiant.

15. Procédé selon l'une quelconque des revendications précédentes,
**caractérisé en ce qu'**
on utilise une colle non résistante à la chaleur, en particulier du polyuréthane-méthacrylate.
